# EUROPÄISCHE PATENTANMELDUNG

(11) **EP 4 167 243 A1**
(43) Veröffentlichungstag der Anmeldung: **19.04.2023**
(21) Anmeldenummer: 22199624.2
(22) Anmeldetag: 04.10.2022
(51) Int. Cl.: G16H 40/00

(54) **BEREITSTELLUNGSSYSTEM FÜR ENDOSKOPE**

(30) Priorität: 13.10.2021 US 202163255047 P
(71) Anmelder: OLYMPUS WINTER & IBE GMBH, 22045 Hamburg (DE)
(72) Erfinder: Siegmund, Ralf, 22926 Ahrensburg (DE); Züwers, Daniel, 22397 Hamburg (DE); Niebuhr, Jan, 24105 Kiel (DE); Ahrens, Christoph Alexander, 21465 Reinbek (DE); Jaskola, Sascha, 22767 Hamburg (DE); Hüber, Mathias, 22299 Hamburg (DE); Tessmann, Ralf, 22149 Hamburg (DE); Schroeder, Stefan, 20459 Hamburg (DE)
(74) Vertreter: Noack, Andreas

(57) **Zusammenfassung**

Es wird ein Bereitstellungssystem für Endoskope in einer Anwendungsumgebung mit einem oder mehreren Untersuchungsräumen (UR1, UR2, UR3) und einem oder mehreren Aufbereitungsplätzen (VR1, VR2, VR3, EDG1, EDG2, TS1, TS2, TS3, AS1, AS2) für Endoskope vorgestellt, wobei das Bereitstellungssystem eingerichtet ist, erste Informationen über vorgesehene Prozeduren zu empfangen, und zweite Informationen über einen Zustand eines oder mehrerer Endoskope zu empfangen, wobei die zweiten Informationen eine Angabe umfassen, ob ein Endoskop einsatzbereit ist, sich momentan in Verwendung befindet, oder momentan aufbereitet wird, wobei das Bereitstellungssystem ferner eine Benutzerschnittstelle (104) mit einer grafischen Benutzeroberfläche (105, 205) umfasst.

Das Bereitstellungssystem zeichnet sich dadurch aus, dass die grafische Benutzeroberfläche (105, 205) einen ersten Wiedergabebereich (110, 210) umfasst, in welchem Daten über einsatzbereite Endoskope angezeigt werden, einen zweiten Wiedergabebereich (120, 220) umfasst, in welchem Daten über geplante Prozeduren angezeigt werden, und einen dritten Wiedergabebereich (130, 230) umfasst, in welchem Daten über Endoskope angezeigt werden, die sich in einem Aufbereitungsprozess befinden.

## Beschreibung

Die Erfindung betrifft ein Bereitstellungssystem für Endoskope.

Endoskope werden seit langem in der Medizin eingesetzt, um schwer zugängliche Körperhöhlen eines Patienten zu untersuchen oder zu behandeln. Sie sind in der Regel mehrfach verwendbar und müssen nach jeder Verwendung einem aufwendigen Aufbereitungsprozess unterzogen werden, bevor sie zur Untersuchung oder Behandlung eines weiteren Patienten eingesetzt werden können.

Ein Aufbereitungsprozess z.B. für gastroenterologische Endoskope umfasst dabei in der Regel eine manuelle Vorreinigung, eine maschinelle Reinigung und Desinfektion, ggf. eine Trocknung, und eine Bereithaltung in einem Vorratsschrank.

Auf endoskopische Prozeduren spezialisierte Praxen oder Abteilungen umfassen regelmäßig eine Vielzahl von Behandlungsräumen, in denen mehrere Prozeduren parallel durchgeführt werden können. Hierzu wird weiterhin eine Vielzahl von Endoskopen unterschiedlichen Typs vorgehalten, für deren Aufbereitung mehrere manuelle und maschinelle Aufbereitungsplätze vorgesehen sind. Im Rahmen einer Belegplanung wird für die einzelnen Behandlungsräume festgelegt, wann welche Prozeduren mit welchen Endoskopen bzw. Endoskoptypen durchzuführen sind, und die jeweils zu behandelnden Patienten werden entsprechend einbestellt. In der Belegplanung kann weiterhin festgelegt werden, an welchen Aufbereitungsplätzen die Endoskope nach deren Nutzung aufbereitet werden.

Bei der Belegplanung müssen einige Parameter zunächst geschätzt werden. Dazu gehören insbesondere die Dauer einzelner Prozeduren und die Dauer der Aufbereitung einzelner Endoskope, bevor diese wieder verwendet werden können. Abweichungen der tatsächlichen Dauer einer Prozedur oder eines Aufbereitungsvorgangs können erhebliche Auswirkungen haben, wenn beispielsweise ein Endoskop aufgrund einer länger andauernden Prozedur nicht rechtzeitig zur Verfügung steht, um nach manueller Vorreinigung zusammen mit weiteren Endoskopen gemeinsam einer maschinellen Reinigung und Desinfektion unterzogen zu werden. In diesem Fall muss entweder der Beginn der maschinellen Reinigung und Desinfektion verzögert werden, wodurch alle betroffenen Endoskope erst später zur erneuten Nutzung zur Verfügung stehen, oder das erst später zur Verfügung stehende Endoskop muss für einen späteren Durchlauf der maschinellen Reinigung und Desinfektion vorgesehen werden, wodurch sich die erneute Bereitstellung des betroffenen Endoskops noch weiter verzögert.

Aus der US 8768721 B2 ist ein System zum Management von Endoskopen bekannt, welches mögliche Auswirkungen eines Ausfalls von Aufbereitungskapazitäten auf die Belegplanung bestimmt und daraus einen zusätzlichen Bedarf an Endoskopen ermittelt, welche dann als Leih- oder Kaufgeräte beschafft werden. Eine kurzfristige Beschaffung zusätzlicher Endoskope ist jedoch nicht immer möglich.

Für eine für die Belegplanung zuständige Person ist es jedoch schwierig, die tatsächlichen Auswirkungen von Verzögerungen einzelner Prozeduren oder Aufbereitungsprozesse auf die aktuelle Belegplanung abzusehen.

Weiterhin erlauben die für die Belegplanung zur Verfügung stehenden Schätzwerte der einzelnen Prozessdauern nur eine begrenzte Genauigkeit bei der Planung.

Es besteht daher eine Aufgabe der Erfindung darin, ein verbessertes Bereitstellungssystem für Endoskope zu entwickeln.

Diese Aufgabe wird gemäß eines ersten Aspekts der Erfindung gelöst durch ein Bereitstellungssystem für Endoskope in einer Anwendungsumgebung mit einem oder mehreren Untersuchungsräumen und einem oder mehreren Aufbereitungsplätzen für Endoskope, wobei das Bereitstellungssystem eingerichtet ist, erste Informationen über vorgesehene Prozeduren zu empfangen, und zweite Informationen über einen Zustand eines oder mehrerer Endoskope zu empfangen, wobei die zweiten Informationen eine Angabe umfassen, ob ein Endoskop einsatzbereit ist, sich momentan in Verwendung befindet, oder momentan aufbereitet wird. Das Bereitstellungssystem umfasst ferner eine Benutzerschnittstelle mit einer grafischen Benutzeroberfläche und ist dadurch weitergebildet, dass die grafische Benutzeroberfläche einen ersten Wiedergabebereich umfasst, in welchem Daten über einsatzbereite Endoskope angezeigt werden, einen zweiten Wiedergabebereich umfasst, in welchem Daten über geplante Prozeduren angezeigt werden, und einen dritten Wiedergabebereich umfasst, in welchem Daten über Endoskope angezeigt werden, die sich in einem Aufbereitungsprozess befinden.

Das Empfangen der zweiten Informationen kann im Sinne der Erfindung umfassen, dass Daten empfangen werden, welche nicht unmittelbar den Zustand eines Endoskops beschreiben, aber Rückschlüsse auf diesen zulassen, und dass diese Daten weiter ausgewertet werden, um den Zustand des Endoskops zu ermitteln. Zusätzlich kann auch ein Aufenthaltsort eines Endoskops ermittelt werden.

Durch die entsprechende Ausführung des Bereitstellungssystems werden die Informationen über die Verfügbarkeit der Endoskope so angeboten, dass ein Benutzer diese intuitiv erfassen kann und sofort erkennt, ob für alle geplanten Prozeduren ein erforderliches Endoskop rechtzeitig bereitstehen wird.

Dabei können der erste Wiedergabebereich, der zweite Wiedergabebereich, und der dritte Wiedergabebereich entlang einer Hauptrichtung angeordnet sein, wobei der dritte Wiedergabebereich zwischen dem ersten Wiedergabebereich und dem zweiten Wiedergabebereich angeordnet ist.

Daten für einzelne Endoskope und/oder Prozeduren können in den Wiedergabebereichen entlang einer zweiten Hauptrichtung angeordnet sein, welche senkrecht zu der ersten Hauptrichtung verläuft. Eine entsprechend rasterförmige Anordnung der Informationen macht diese für einen Benutzer besonders schnell erfassbar.

In einer bevorzugten Ausführung eines Bereitstellungssystems gemäß der Erfindung können die Daten in dem ersten Wiedergabebereich, dem zweiten Wiedergabebereich, und dem dritten Wiedergabebereich so angeordnet sein, dass Daten zu einem Endoskop, die im ersten Wiedergabebereich oder im dritten Wiedergabebereich dargestellt sind, entlang der zweiten Hauptrichtung mit Daten einer Prozedur im zweiten Wiedergabebereich fluchten, in welcher das jeweilige Endoskop verwendet werden soll. Die Erfassung miteinander in Beziehung stehenden Informationen wird hierdurch noch weiter vereinfacht.

In einer möglichen Ausführung eines Bereitstellungssystems nach der Erfindung kann im dritten Wiedergabebereich für jedes in einem Aufbereitungsprozess befindliche Endoskop eine Visualisierung dargestellt werden, welche den Fortschritt des Aufbereitungsprozesses wiedergibt.

Die Visualisierung kann für jeden Schritt eines mehrere Schritte umfassenden Aufbereitungsprozesses eine Visualisierungselement umfassen, welches anzeigt, ob der jeweilige Schritt abgeschlossen, in Arbeit, oder ausstehend ist.

Im dritten Wiedergabebereich kann für jedes in einem Aufbereitungsprozess befindliche Endoskop ein voraussichtlicher Zeitpunkt angezeigt werden, zu dem das Endoskop verfügbar sein wird.

In einer weiteren bevorzugten Ausführung eines Bereitstellungssystems nach der Erfindung kann dieses eingerichtet sein, bei der Ermittlung eines Zeitpunktes, zu dem ein Endoskop voraussichtlich verfügbar sein wird, historische Daten vorausgegangener Aufbereitungsprozesse zu berücksichtigen. Auf diese Weise kann der Zeitpunkt besonders zuverlässig ermittelt werden.

Das Bereitstellungssystem kann dazu eigerichtet sein, die Dauer von durchgeführten Aufbereitungsprozessen zu speichern und/oder statistisch auszuwerten.

Gemäß eines weiteren Aspekts der Erfindung wird die Aufgabe gelöst durch ein Verfahren zum Betrieb eines Bereitstellungssystems für Endoskope, mit den Schritten: Empfangen erster Informationen über geplante Prozeduren, Empfangen zweiter Informationen über einen Zustand eines oder mehrerer Endoskope, wobei die zweiten Informationen eine Angabe umfassen, ob ein Endoskop einsatzbereit ist, sich momentan in Verwendung befindet, oder momentan aufbereitet wird, und Anzeigen der ersten und zweiten Informationen in einer grafischen Benutzeroberfläche.Das Verfahren ist dadurch weitergebildet, dass die grafische Benutzeroberfläche einen ersten Wiedergabebereich umfasst, in welchem Daten über einsatzbereite Endoskope angezeigt werden, einen zweiten Wiedergabebereich umfasst, in welchem Daten über geplante Prozeduren angezeigt werden, und einen dritten Wiedergabebereich umfasst, in welchem Daten über Endoskope angezeigt werden, die sich in einem Aufbereitungsprozess befinden.

Bezüglich möglicher Weiterbildungen sowie der dadurch erreichbaren Vorteile und Wirkungen wird auf das oben gesagte ausdrücklich verwiesen.

Im Folgenden wird ein Aufbereitungssystem für Endoskope gemäß der Erfindung anhand einiger beispielhafter Figuren näher beschrieben. Dabei dient die Darstellung nur dem besseren Verständnis der Erfindung, ohne diese einzuschränken.

Es zeigen:
Figur 1: ein Bereitstellungssystem für Endoskope,
Figur 2: eine Benutzeroberfläche,
Figur 3: eine weitere Benutzeroberfläche,
Figur 4: ein Kennlinienfeld.

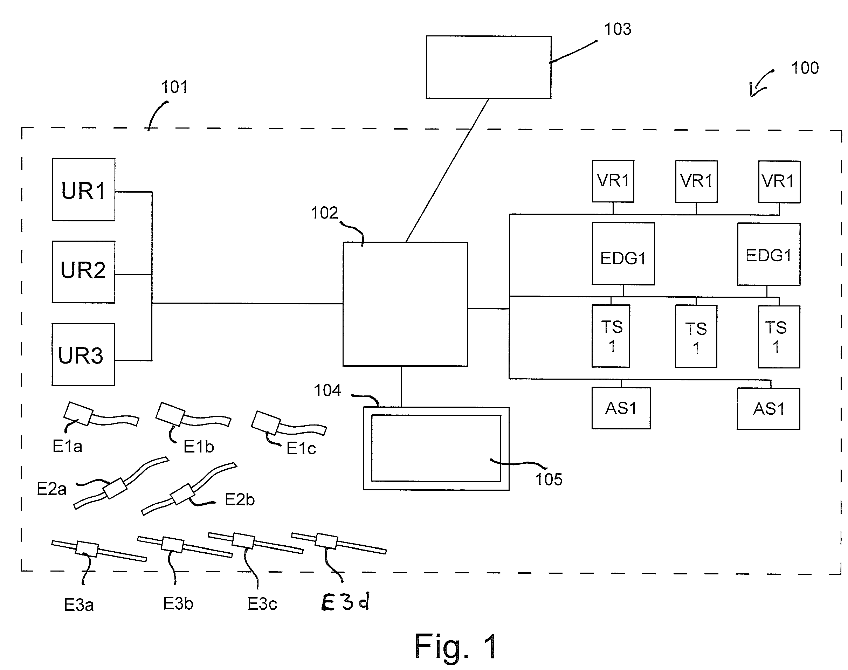

Figur 1 zeigt ein Bereitstellungssystem 100 für Endoskope in einer beispielhaften Anwendungsumgebung 101, welche eine ambulante Endoskopiepraxis oder eine Endoskopieabteilung eines Krankenhauses sein kann.

Die Anwendungsumgebung umfasst mehrere Untersuchungsräume UR1, UR2, UR3. Die Anzahl der Untersuchungsräume kann dabei beliebig variieren. Der Begriff "Untersuchungsraum" schließt nicht aus, dass in dieser Räumen auch Prozeduren mit interventionellen Anteilen wie Biopsien oder Verödungen durchgeführt werden.

Weiterhin umfasst die Anwendungsumgebung eine Aufbereitungsstation zur Aufbereitung genutzter Endoskope, welche manuelle Vorreinigungsplätze VR1, VR2, VR3, Endoskop-Aufbereitungsgeräte EDG1, EDG2, Trockenschränke TS1, TS2, TS3, und Aufbewahrungsschränke AS1, AS2 umfasst. Die Anzahlen der einzelnen Elemente ist wieder beliebig und dient nur als Beispiel.

Zur Durchführung von endoskopischen Untersuchungen in den Untersuchungsräumen UR1, UR2, UR3 werden mehrere Endoskope verschiedenen Typs vorgehalten. Beispielsweise werden Endoskope E1a, E1b, E1c eines ersten Typs E1, Endoskope E2a, E2b eines zweiten Typs E2, und Endoskope E3a, E3b, E3c, E3d eines dritten Typs E3 vorgehalten. Bei den Endoskoptypen E1, E2, und E3 kann es sich um Gastroskope, Bronchoskope, und Colonoskope handeln. Natürlich können auch Endoskope anderer Typen vorhanden sein.

Das Bereitstellungssystem 100 umfasst eine Datenverarbeitungsanlage 102, welche eingerichtet ist, Informationen über geplante und/oder laufende Prozeduren und Aufbereitungsprozesse sowie ggf. über den aktuellen Aufenthaltsort und den Zustand der einzelnen Endoskope zu empfangen und zu verarbeiten.

Informationen über vorgesehen Prozeduren können der Datenverarbeitungsanlage 102 beispielsweise von einem Krankenhaus- oder Praxisverwaltungssystem 103 zur Verfügung gestellt werden. Die kann über eine Schnittstelle erfolgen, z.B. mittels des DICOM-Standards. Solche Informationen können insbesondere die Art der Prozedur, den geplanten Beginn der Prozedur, sowie den Untersuchungsraum umfassen, in welchem die Prozedur durchgeführt werden soll.

Informationen über laufende Aufbereitungsprozesse werden in der Regel von den Endoskop-Aufbereitungsgeräten bereitgestellt.

Zur Ermittlung des Aufenthaltsorts der einzelnen Endoskope sind in den Untersuchungsräumen UR1, UR2, UR3 sowie an den Aufbereitungsstationen Sensoren vorgesehen, welche Identifikationsmerkmale von Endoskopen erfassen, die sich in dem Untersuchungsraum bzw. an der Aufbereitungsstation befinden. Solche Sensoren können z.B. RFID-Lesegeräte umfassen, welche den Endoskopen individuell zugeordnete RFID-Tags automatisch oder manuell gesteuert erfassen.

Informationen über den aktuellen Zustand der einzelnen Endoskope ergeben sich größtenteils aus den oben genannten Angaben. Befindet sich ein Endoskop beispielsweise in einem Untersuchungsraum, so kann als Zustand angenommen werden, dass sich das Endoskop in Verwendung befindet. Befindet sich das Endoskop hingegen an einem manuellen Vorreinigungsplatz, so kann als Zustand angenommen werden, dass das Endoskop gerade manuell vorgereinigt wird. Dabei kann zusätzlich erfasst werden, seit wann sich das Endoskop in der Vorreinigung befindet.

Die Endoskop-Aufbereitungsgeräte EDG1, EDG2 liefern in der Regel zusätzlich Informationen über die Art und den Fortschritt des gewählten Aufbereitungsprogramms, welche ebenfalls den Zustand der Endoskope angeben, die sich in dem jeweiligen Endoskop-Aufbereitungsgerät befinden.

Aus den empfangenen Daten ermittelt das Bereitstellungssystem 100, ob für die geplanten Prozeduren jeweils geeignete Endoskope bereitstehen. Dazu werden jeder geplanten Prozedur ein oder mehrere kompatible Endoskoptypen zugeordnet, mit denen die Prozedur durchgeführt werden kann. Gleichzeitig wird die von jedem Endoskoptyp momentan zur Verfügung stehenden Anzahl von Endoskopen ermittelt. Dabei wird ein Endoskop als zur Verfügung stehend gewertet, wenn es vollständig aufbereitet ist und seine maximal zulässige Lagerdauer in einem Aufbewahrungsschrank nicht überschritten hat.

Neben den momentan zur Verfügung stehenden Endoskopen können bei der Verfügbarkeitsprüfung solche Endoskope zu berücksichtigen sein, die sich momentan in Benutzung oder in der Aufbereitung befinden, aber bis zu einem geplanten Beginn einer Prozedur vollständig aufbereitet und somit verfügbar sein werden. Hierbei werden bekannte Dauern von Prozeduren und einzelnen Aufbereitungsprozessen genutzt, um eine möglichst genaue Vorhersage treffen zu können.

### Dashboard

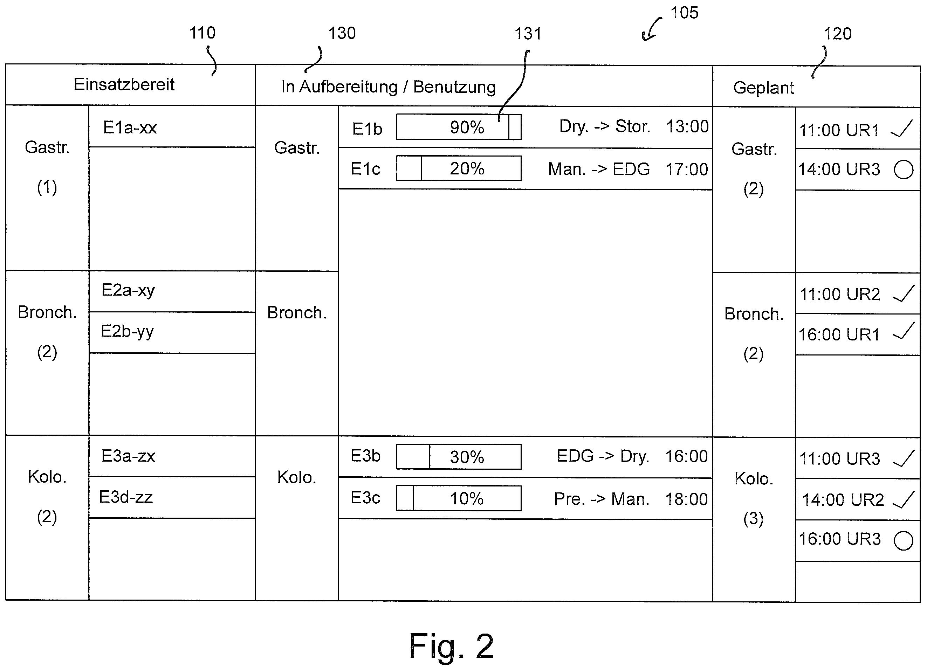

Das Bereitstellungssystem 100 umfasst weiterhin eine Benutzerschnittstelle 104, über welche die ermittelten Informationen über die Verfügbarkeit von Endoskopen angezeigt werden. Ein wesentliches Element der Benutzerschnittstelle 104 ist eine grafische Benutzeroberfläche 105, in welcher die Informationen intuitiv dargestellt werden. Diese Benutzeroberfläche 105 wird auch als "Dashboard" bezeichnet. Eine mögliche Ausführung der Benutzeroberfläche 105 ist in Figur 2 dargestellt.

Die Benutzeroberfläche 105 weist einen ersten Wiedergabebereich 110 auf, in welchem Daten über die momentan verwendungsbereit zur Verfügung stehenden Endoskope angezeigt werden. In einem zweiten Wiedergabebereich 120 werden Daten über geplante Prozeduren angezeigt. In einem dritten Wiedergabebereich 130 werden Daten über derzeit in der Aufbereitung befindlichen Endoskope angezeigt.

Der erste Wiedergabebereich 110, der zweite Wiedergabebereich 120, und der dritte Wiedergabebereich 130 sind entlang einer Hauptrichtung, im dargestellten Beispiel entlang einer Horizontale, angeordnet. Dabei ist der dritte Wiedergabebereich 130 vorzugsweise zwischen dem ersten Wiedergabebereich und dem dritten Wiedergabebereich angeordnet.

In den Wiedergabebereichen 110, 120, 130 sind Daten bezüglich einzelner Endoskope und/oder Prozeduren entlang einer zweiten Hauptrichtung angeordnet, die vorzugsweise senkrecht zur ersten Hauptrichtung verläuft. Im dargestellten Beispiel ist die zweite Hauptrichtung eine Vertikale.

### Erster Wiedergabebereich

Zur Anzeige der verfügbaren Endoskope im ersten Wiedergabebereich 110 können die Typbezeichnungen und/oder Seriennummern der verfügbaren Endoskope aufgelistet sein. Die Anzeige kann nach Endoskoptypen sortiert und/oder gruppiert sein. Eine zusätzliche Gruppierung kann nach der Art der Prozedur erfolgen, für welche die jeweiligen Endoskope geeignet sind. Für die so gebildeten Gruppen können die Anzahl der in jeder Gruppe verfügbaren Endoskope ausgegeben werden, um eine besonders schnelle Erfassung der relevanten Information durch einen Betrachter zu ermöglichen.

Die anzuwendenden Sortierungen und/oder Gruppierungen können durch den Benutzer konfiguriert werden. Ebenso kann der Benutzer definieren, welche Informationen im ersten Wiedergabebereich angezeigt werden sollen. Beispielsweise kann die Anzeige der Seriennummern verfügbarer Endoskope aktiviert oder deaktiviert werden. Als weitere Information kann eine verbleibende Lagerdauer für jedes verfügbare Endoskop angezeigt werden.

Im dargestellten Beispiel wird im ersten Wiedergabebereich 110 angezeigt, dass ein Gastroskop verfügbar ist, nämlich das Gastroskop E1a mit der Seriennummer E1a-xx. Weiterhin sind zwei Bronchoskope verfügbar, nämlich die Bronchoskope E2a, E2b mit den Seriennummern E2a-xy und E2b-yy. Zusätzlich sind zwei Koloskope E3a, E3d mit den Seriennummern E3a-zy und E3d-zz.

### Zweiter Wiedergabebereich

Im zweiten Wiedergabebereich 120 werden die geplanten Prozeduren angezeigt. Die Anzeige kann beispielsweise die Art der Prozedur, den geplanten Beginn, und den Untersuchungsraum umfassen. Ähnlich wie im ersten Wiedergabebereich kann auch im zweiten Wiedergabebereich eine Sortierung und/oder Gruppierung erfolgen, z.B. nach Art der geplanten Prozedur. Auch hier kann zur schnellen Erfassung der Information eine Anzahl von geplanten Prozeduren für jede Gruppe von Prozeduren angezeigt werden.

Auch für den zweiten Wiedergabebereich kann ein Benutzer Art und Umfang der angezeigten Informationen konfigurieren. Beispielsweise kann ein Benutzer über ein Filter steuern, für welchen Zeitraum geplante Prozeduren angezeigt werden, und/oder nur bestimmte Arten von Prozeduren zur Anzeige auswählen.

Im dargestellten Beispiel wird im zweiten Wiedergabebereich 120 angezeigt, dass zwei Gastroskopien geplant sind, nämlich um 11:00 Uhr in Raum UR1 und um 14:00 Uhr in UR3. Weiterhin sind zwei Bronchoskopien geplant, nämlich um 11:00 Uhr in Raum UR2 und um 16:00 Uhr in Raum UR1. Schließlich sind noch drei Koloskopien geplant, nämlich um 11:00 Uhr in Raum UR3, um 14:00 Uhr in Raum UR2 und um 16:00 Uhr in Raum UR3.

### Dritter Wiedergabebereich

Im dritten Wiedergabebereich 130 werden die gerade im Aufbereitungsprozess befindlichen Endoskope angezeigt. Hierbei kann beispielsweise eine verbleibende Dauer des Aufbereitungsprozesses und/oder der prognostizierte Zeitpunkt der Verfügbarkeit angezeigt werden. Zusätzlich kann der Aufbereitungsschritt angezeigt werden, in dem sich das Endoskop gerade befindet, sowie die verbleibende Dauer des Aufbereitungsschrittes. Weiterhin kann eine Visualisierung 131 in Form einer Prozentzahl und/oder eines Fortschrittsbalkens dazu dienen, den Fortschritt des Aufbereitungsprozesses schnell und eindeutig zu erfassen.

Zusätzlich zu den Endoskopen, die sich momentan im Aufbereitungsprozess befinden, können im dritten Wiedergabebereich auch solche Endoskope angezeigt werden, welche sich gerade in Verwendung befinden.

Ähnlich wie im ersten und zweiten Wiedergabebereich können die im dritten Wiedergabebereich angezeigten Endoskope nach Endoskoptyp und/oder Prozedurtyp sortiert und/oder gruppiert werden. Auch hier kann die Anzahl von Endoskopen in jeder Gruppe zur schnellen Erfassung angezeigt werden.

Im dargestellten Beispiel befinden sich gerade die Gastroskope E1b und E1c in der Aufbereitung, wobei die Aufbereitung des Gastroskops E1b zu 90% abgeschlossen ist, so dass das Gastroskop E1b um 13:00 Uhr verwendungsbereit sein wird. Das Gastroskop E1b befindet sich dabei momentan im Trocknungsprozess und wartet auf die Überführung in die Aufbewahrung. Die Aufbereitung des Gastroskops E1c ist zu 20% abgeschlossen, es wird um 17:00 Uhr einsatzbereit sein. Das Gastroskop E2c befindet sich momentan in der manuellen Vorreinigung und wartet auf die Überführung in ein Endoskop-Aufbereitungsgerät.

Die Anzeige der Informationen über die Verfügbarkeit von Endoskopen in den beschriebenen drei Wiedergabebereichen ermöglicht es einem Benutzer des Bereitstellungssystems, mit einem einzigen Blick zu erfassen, ob für alle im ausgewählten Zeitraum geplanten Prozeduren die erforderlichen Endoskope bereitstehen bzw. rechtzeitig bereitstehen werden.

So ist z.B. sofort erkennbar, dass für die um 11:00 Uhr geplante Gastroskopie das Gastroskop E1a bereitsteht, und dass das für die um 14:00 Uhr geplante Gastroskopie benötigte Gastroskop E1b bereits um 13:00 Uhr fertig aufbereitet sein wird.

Für die geplanten Bronchoskopien um 11:00 und 16:00 Uhr sind die Bronchoskope E2a und E2b einsatzbereit.

Für die Koloskopien um 11:00 und 14:00 Uhr sind die Koloskope E3a und E3d einsatzbereit. Weiterhin ist sofort ersichtlich, dass das Koloskop E3b um 16:00 Uhr einsatzbereit sein wird, um in der für 16:00 Uhr geplanten Koloskopie verwendet zu werden.

Um die schnelle optische Erfassung zu unterstützen, können die Informationen in den jeweiligen Wiedergabebereichen farblich hinterlegt oder mit Piktogrammen versehen werden. So können beispielsweise im zweiten Wiedergabebereich 120 solche geplanten Prozeduren, für die einsatzbereite Endoskope bereitstehen, grün hinterlegt und/oder mit einem "Haken"-Symbol versehen sein. Prozeduren, für welche die Endoskope gerade aufbereitet werden, aber rechtzeitig zur Verfügung stehen sollen, können gelb hinterlegt und/oder mit einem "Kreis"-Symbol versehen sein. Prozeduren, für die ein Endoskop voraussichtlich nicht rechtzeitig zur Verfügung stehen wird, können rot hinterlegt und/oder mit einem "Dreieck"-Symbol versehen sein.

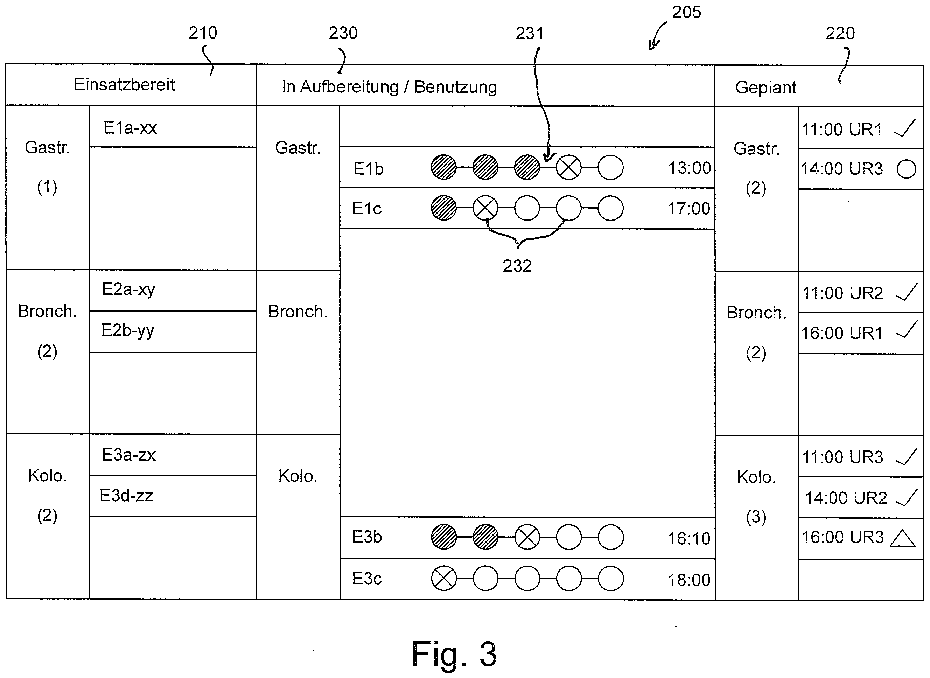

In Figur 3 ist eine weitere mögliche Ausführungsform der Benutzeroberfläche 205 dargestellt. Sie umfasst wiederum einen ersten Wiedergabebereich 210, einen zweiten Wiedergabebereich 220, und einen dritten Wiedergabebereich 230. Dabei sind der erste Wiedergabebereich 210 und der zweite Wiedergabebereich 220 so aufgebaut wie die entsprechenden Wiedergabebereiche 110, 120 der Benutzeroberfläche 105. Der dritte Wiedergabebereich 230 unterscheidet sich hingegen im Aufbau von dem dritten Wiedergabebereich 130.

Im dritten Wiedergabebereich 230 ist der Aufbereitungsfortschritt der angezeigten Endoskope durch eine Visualisierung 231 in Form einer "Perlenschnur" mit mehreren Visualisierungselementen 232, im dargestellten Beispiel als Knoten dargestellt, wobei jeder Knoten für einen bestimmten Schritt des Aufbereitungsprozesses steht. Dabei sind die Knoten unterschiedlich dargestellt, abhängig davon, ob der entsprechende Aufbereitungsschritt noch aussteht, gerade durchgeführt wird, oder bereits abgeschlossen ist. So können abgeschlossene Schritte mit einem ausgefüllten Knoten und ausstehende Schritte mit einem umrandeten Knoten dargestellt sein, während gerade ausgeführte Schritte beispielsweise mit einem Kreuz dargestellt werden können. Andere Darstellungen sind natürlich ebenso möglich.

Ähnlich wie im dritten Wiedergabebereich 130 der Figur 2 wird auch im dritten Wiedergabebereich 230 der Figur 3 die Uhrzeit angezeigt, zu welcher das entsprechende Endoskop einsatzbereit sein wird.

Als eine weitere Besonderheit ist die Darstellung der in Aufbereitung befindlichen Endoskope im dritten Wiedergabebereich 230 so weit nach unten verschoben, dass sie unterhalb der Darstellung der einsatzbereiten Endoskope im ersten Wiedergabebereich 210 erfolgt. Durch diese Verschiebung werden, bei entsprechender Sortierung der Anzeigen, die in Aufbereitung befindlichen Endoskope im dritten Wiedergabebereich 230 in einer Höhe mit den geplanten Prozeduren im zweiten Wiedergabebereich 220 angezeigt, in welchen das jeweilige Endoskop verwendet werden soll. Die miteinander in Beziehung stehenden Daten in den Wiedergabebereichen 210, 220, 230 fluchten somit in der zweiten Hauptrichtung, im Beispiel der Vertikalen. Auf diese Weise kann ein Benutzer des Bereitstellungssystems 100 noch leichter erkennen, ob für jede geplante Prozedur rechtzeitig ein geeignetes Endoskop bereitstehen wird.

In dem in Figur 3 dargestellten Beispiel hat sich die Bereitstellung des Koloskops E3b gegenüber der in Figur 2 dargestellten Situation um 10 Minuten verzögert. Solche Verzögerungen können z.B. auftreten, wenn Schwankungen im Wasserdruck oder in der Wassertemperatur der Wasserversorgung dazu führen, dass ein Dosier- oder Heizvorgang in einem Endoskop-Aufbereitungsgerät länger dauert als geplant. Die für 16:00 Uhr geplante Koloskopie ist daher mit einem "Dreieck"-Symbol versehen, um zu kennzeichnen, dass hier eine Anpassung der Belegplanung erforderlich sein kann.

Um die Vorhersagegenauigkeit zu erhöhen, können weitere Informationen herangezogen werden.

### Dauer und Fortschritt einer Prozedur

Die durchschnittliche Dauer bestimmter Standardprozeduren wie z.B. einer Gastroskopie oder einer Koloskopie ist recht gut bekannt. Bei der Ermittlung der zu erwartenden Dauer einer Prozedur können zusätzlich historische Daten berücksichtigt werden. Beispielsweise können die Dauern vorangegangener Untersuchungen des selben Patienten herangezogen werden, ebenso solche Untersuchungen anderer Patienten, die vom selben Arzt und/oder im selben Untersuchungsraum durchgeführt wurden. Hierdurch lässt sich die Vorhersagegenauigkeit für die Dauer der Untersuchung deutlich steigern.

Zu diesem Zweck werden die Dauern zurückliegender Prozeduren in einer Datenbank gespeichert, welche in der Datenverarbeitungsanlage 102 implementiert ist. Dabei können persönliche Daten bezüglich des Patienten oder des Arztes auf geeignete Weise anonymisiert werden.

Um nun zu ermitteln, wann eine aktuelle Prozedur beendet sein wird, so dass das verwendete Endoskop in die Aufbereitung überführt werden kann, kann das Bereitstellungssystem verschiedene Filter auf die gespeicherten Daten anwenden, und die gefilterten Daten dann statistisch auswerten.

Als Filter kommen hier vor allem die Art der Prozedur und der durchführende Arzt in Betracht. Aus den so gefilterten Daten kann dann ein Mittelwert der Prozedurdauer bestimmt werden. Alternativ oder zusätzlich kann eine Trendanalyse durchgeführt werden, durch welche z.B. eine Lernkurve eines Arztes berücksichtigt wird, während der sich die Dauer der Prozeduren langsam verringert.

Allerdings kann es auf Grund von unterschiedlichen anatomischen Gegebenheiten und/oder Komplikationen zu Abweichungen von der statistisch ermittelten Dauer kommen. Um solche Abweichungen frühzeitig zu erkennen und deren Auswirkung auf die Verfügbarkeitsplanung von Endoskopen zu ermitteln, kann während der Prozedur laufend der Fortschritt ermittelt und an das Bereitstellungssystem übermittelt werden.

Endoskopische Standardprozeduren sind üblicherweise in vorgegebene Abschnitte unterteilt, welche beispielsweise durch das Erreichen bestimmter anatomischer Landmarken definiert sind. Das Erreichen einer solchen Landmarke wird in der Regel durch das Abspeichern eines Standbildes der anatomischen Landmarke dokumentiert.

Anhand der bereits abgespeicherten Standbilder kann somit abgeschätzt werden, wie weit die Prozedur fortgeschritten ist. Abweichungen von einem durchschnittlichen Prozedurfortschritt lassen sich so frühzeitig erkennen und können bei der Verfügbarkeitsplanung berücksichtigt werden.

### Manuelle Vorreinigung

Vor der maschinellen Aufbereitung müssen Endoskope manuell vorgereinigt werden, um grobe Verschmutzungen zu entfernen. Die Dauer der Vorreinigung ist hauptsächlich durch vorgegebene Protokolle vorgegeben, kann aber auch in Abhängigkeit von der jeweils durchführenden Fachkraft und oder des verwendeten Vorreinigungsplatzes schwanken.

Sobald ein Endoskop an einem Vorreinigungsplatz registriert wird, steht dem Bereitstellungssystem die Information zur Verfügung, welche Fachkraft für die Vorreinigung verantwortlich ist. Das Bereitstellungssystem kann dann anhand von historischen Daten abschätzen, wie lange die Vorreinigung dauern wird. Dazu kann beispielsweise über einen längeren Zeitraum hinweg ermittelt werden, wie lange die jeweilige Fachkraft für die Vorreinigung des jeweiligen Endoskoptyps gebraucht hat. Ggf. können auch detailliertere Abschätzungen vorgenommen werden, welche einen Wochentag oder eine Tageszeit berücksichtigen. Ebenso können lediglich solche Vorreinigungsprozesse für die Auswertung ausgewertet werden, welche am selben Vorreinigungsplatz ausgeführt wurden.

Die Ermittlung erfolgt auch hier durch Filterung und statistische Auswertung gespeicherter Daten über zurückliegende Vorgänge.

### Bestückung von Endoskop-Aufbereitungsgeräten

Moderne Endoskop-Aufbereitungsgeräte sind in der Regel zur Aufbereitung mehrerer Endoskope in einem Arbeitsgang vorgesehen. Dadurch, dass mehrere Endoskope gleichzeitig aufbereitet werden, übertragen sich auch Verzögerungen der Bereitstellung einzelner Endoskope auf andere Endoskope, welche gleichzeitig mit dem verspäteten Endoskop aufbereitet werden sollen.

Eine solche Übertragung von Verzögerungen kann durch das Bereitstellungssystem erkannt und bei der Verfügbarkeitsplanung berücksichtigt werden.

### Fortschritt der maschinellen Aufbereitung

Die Prozessdauer der maschinellen Aufbereitung ist im Wesentlichen durch feste Aufbereitungsprogramme vorgegeben. Trotzdem kann es hierbei zu zeitlichen Abweichungen kommen.

Beispielsweise führen Endoskop-Aufbereitungsgeräte üblicherweise einen Dichtigkeitstest für die aufzubereitenden Endoskope durch. Hierzu werden die Endoskope mit einem Druckgas beaufschlagt, und der Druckverlauf wird überwacht. Da sich die Förderleistung einer für den Druckaufbau verwendeten Pumpe mit der Zeit ändern kann, kann es zu schleichenden Änderungen der Dauer des Dichtigkeitstests kommen.

Weitere Gründe für Abweichungen können Wasserdruckschwankungen oder Änderungen der Wassertemperatur sein, welche zu veränderten Dosier- oder Aufheizzeiten führen. Solche Variationen können zufällig oder systematisch sein. Ebenso können schleichende Veränderung der Förder- oder Heizleistung einzelner Aggregate einer Endoskop-Aufbereitungsmaschine Ursache für Veränderungen der Prozessdauer sein.

Neuere Generationen von Endoskop-Aufbereitungsmaschinen stellen Informationen über den Prozessfortschritt über eine Datenschnittstelle zur Verfügung, so dass diese Informationen direkt durch das Bereitstellungssystem berücksichtigt werden können.

Ältere Endoskop-Aufbereitungsmaschinen stellen entsprechende Fortschrittsinformationen ggf. nicht zur Verfügung. Hier kann trotzdem eine recht genaue Abschätzung des Prozessfortschritts erfolgen, wenn beispielsweise historische Daten ausgewertet werden, wie lange ein bestimmtes Aufbereitungsprogramm an einer bestimmten Endoskop-Aufbereitungsmaschine üblicherweise andauert. Hierdurch können systematische Abweichungen von einer gewöhnlichen Prozessdauer ebenfalls vorhergesehen und durch das Bereitstellungssystem berücksichtigt werden. Die Auswertung der historischen Daten erfolgt ähnlich wie bezüglich der Prozedurdauer oder der Vorreinigung beschrieben.

### Trocknung und Lagerung

Nach der maschinellen Aufbereitung werden Endoskope zumeist zur Trocknung in Trockenschränke verbracht und dort unter kontrollierten Umgebungsbedingungen getrocknet. Die Dauer des Trocknungsprozesses ist kaum Schwankungen unterworfen. Nach der Trocknung werden Endoskope üblicherweise in Aufbewahrungsschränke überführt, wo sie ebenfalls unter kontrollierten Umweltbedingungen bis zu ihrer nächsten Verwendung gelagert werden. Dabei ist die maximal zulässige Lagerdauer begrenzt, nach Überschreiten der Lagerdauer muss ein Endoskop ggf. neu aufbereitet werden.

### Maßnahmen bei Abweichungen

Wenn die Auswertung der Verfügbarkeit der Endoskope Hinweise dafür bietet, dass für eine geplante Untersuchung nicht rechtzeitig ein geeignetes Endoskop bereitsteht, so müssen ggf. Maßnahmen ergriffen werden, um dem entgegenzuwirken.

In einem einfachen Fall wie der in Figur 3 dargestellten Situation, dass ein Endoskop um einigen Minuten verspätet zur Verfügung stehen wird, kann die geplante Prozedur etwas verschoben werden. Hierzu muss ggf. lediglich eine Information an den Patienten gegeben werden, um diesen über die Verschiebung zu informieren.

Allerdings kann in ungünstigen Fällen eine Verzögerung einer Prozedur zu einer Kette von weiteren Verzögerungen führen, welche Unmut bei den Patienten und dem Personal auslösen können. Es kann daher hilfreich sein, weitere mögliche Gegenmaßnahmen gegen eine Verzögerung vorzusehen. Das Bereitstellungssystem 100 ist aus diesem Grund eingerichtet, eine dynamische Anpassung von Aufbereitungsprozessen für Endoskope zu ermöglichen.

Üblicherweise sind die während der Aufbereitung eines Endoskops durchzuführenden Schritte durch Protokolle fest vorgegeben. Diese Protokolle schreiben unter anderem vor, wie lange Kanäle eines Endoskops während der Vorreinigung mit einer Bürste zu reinigen sind, welche Einwirkzeiten, Wirkstoffkonzentrationen, und Prozesstemperaturen bei der maschinellen Aufbereitung einzuhalten sind, und unter welchen Umgebungsbedingungen ein Endoskop zu trocknen ist. Die entsprechenden Protokolle sind in der Regel vom Hersteller der Endoskope vorgegeben, um eine wirksame Aufbereitung zu gewährleisten.

Die Wirksamkeit der Aufbereitung wird üblicherweise durch die erreichte Reduktion der Anzahl von koloniebildenden Mikroorganismen (CFU, "Colony Forming Units") definiert. Dabei bewirkt jeder Schritt in einem vorgegebenen Aufbereitungsprotokoll eine bestimmte Reduktion der CFU, und die einzelnen Reduktionen addieren bzw. multiplizieren sich zu der insgesamt erreichten Reduktion (Die Reduktion wird zumeist logarithmisch angegeben, z.B. in einer Anzahl von Zehnerpotenzen, um welche die Anzahl der CFU reduziert wird).

Das Bereitstellungssystem 100 weist einen Regelspeicher auf, in welchem Regeln hinterlegt sind, nach denen die Prozessparameter der vorgegebenen Aufbereitungsschritte verändert werden können, ohne dass die Wirksamkeit der Aufbereitungsprozesse beeinträchtigt wird. Der Regelspeicher kann Bestandteil der Datenverarbeitungsanlage 102 sein.

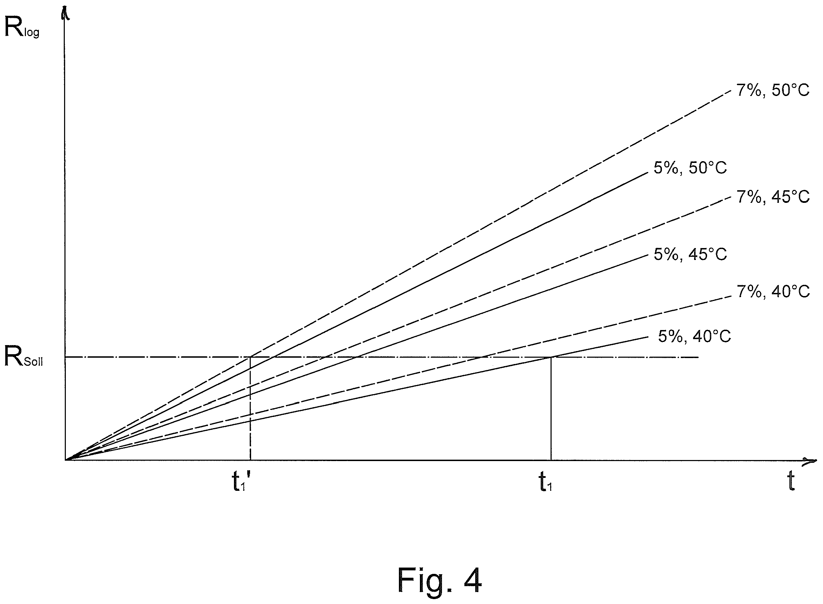

Beispielsweise kann in dem Regelspeicher ein Zusammenhang zwischen einzelnen Prozessparametern eines einzelnen Aufbereitungsschritts und dessen Wirksamkeit in Form eines Kennlinienfeldes hinterlegt sein. Ein solches Kennlinienfeld ist exemplarisch in Figur 4 dargestellt.

Figur 4 zeigt den Zusammenhang zwischen einer Reduktion der CFU in einem Desinfektionsschritt in einer Endoskop-Aufbereitungsmaschine. Die Reduktion R ist logarithmisch als R_{log} auf der Hochachse des Kennlinienfeldes aufgetragen. Auf der Längsachse ist die Prozessdauer t linear aufgetragen.

In dem Desinfektionsschritt wird das Endoskop für die Prozessdauer t bei vorgegebener Temperatur mit einer Desinfektionslösung mit vorgegebener Konzentration beaufschlagt. Die Kennlinien in Figur 4 zeigen an, wie der Verlauf der Reduktion der CFU bei unterschiedlichen Konzentrationen und Temperaturen ist. Durchgezogene Linien zeigen den zeitlichen Verlauf der Reduktion der CFU bei einer Konzentration der Desinfektionslösung von 5%, und bei Temperaturen von 40°C, 45°C, und 50°C. Gestrichelte Linien zeigen den Verlauf der Reduktion bei einer Konzentration von 7%, ebenfalls bei Temperaturen von 40°C, 45°C, und 50°C. Es ist erkennbar, dass eine vorgegebene Reduktion von Rₛₒₗₗ nach einer regulären Prozessdauer von t₁ erreicht ist, wenn eine Konzentration von 5% und eine Temperatur von 40°C eingestellt sind. Hingegen wird die selbe Reduktion Rₛₒₗₗ schon nach wesentlich kürzerer Prozessdauer t₁' erreicht, wenn eine Konzentration von 7% und eine Temperatur von 50°C eingestellt sind.

Das in Figur 4 dargestellte Kennlinienfeld dient nur als ein Beispiel. Ähnliche Kennlinienfelder können für andere Aufbereitungsschritte aufgestellt werden.

Im Falle einer Verzögerung der Bereitstellung eines benötigten Endoskops, wie in Figur 3 für das Endoskop E3b dargestellt, kann das Bereitstellungssystem 100 eingerichtet sein, Maßnahmen vorzuschlagen, um die Auswirkungen der Verzögerung zu kompensieren oder zu reduzieren.

Dazu kann ein das Bereitstellungssystem ein Computerprogramm ausführen, welches die im Regelspeicher abgelegten Kennlinienfelder daraufhin untersucht, ob ein für das betroffene Endoskop noch auszuführender Aufbereitungsschritt so modifiziert werden kann, dass die Verzögerung ausgeglichen wird. Hierbei werden vorzugsweise Sicherheitsgrenzen einzelner Parameter einzuhalten sein, um eine Beschädigung des Endoskops zu vermeiden.

Identifizierte Möglichkeiten für eine Anpassung des Aufbereitungsprozesses können einem Benutzer des Bereitstellungssystems zur Auswahl angeboten werden. Der Benutzer kann dann z.B. aufgrund weiterführender Überlegungen entscheiden, ob eine Modifikation durchgeführt wird oder nicht. Dabei kann der Benutzer wirtschaftliche Auswirkungen der Modifikation, wie z.B. eine erhöhte Abnutzung des Endoskops, gegen die Auswirkungen der Verzögerung, wie z.B. Störung der geplanten Prozeduren, abwägen.

Neben einer separaten Modifikation einzelner Aufbereitungsschritte, bei denen die Reduktion der CFU für jeden Aufbereitungsschritt beibehalten wird, können auch aufeinander folgende Aufbereitungsschritte so modifiziert werden, dass eine Änderung der Reduktion bei einem Schritt durch eine entgegengesetzte Änderung der Reduktion bei einem anderen Schritt kompensiert wird.

Die Ermittlung und Anbietung von Anpassungen kann durch den Benutzer durch Aktivierung einer Schaltfläche in der grafischen Benutzeroberfläche ausgelöst werden. Hierzu kann beispielsweise ein Datenelement im zweiten Wiedergabereich 120, 220, welches die von einer Verzögerung betroffene Prozedur repräsentiert, als interaktive Schaltfläche ausgeführt sein, deren Betätigung durch ein grafisches Eingabegerät wie eine Maus, oder durch Berührung bei Darstellung der Benutzeroberfläche auf einem berührungsempfindlichen Bildschirm, die Ermittlung auslöst. Ermittelte Anpassungen können dann in Form einer Liste, z.B. in einem "pop-up" Fenster, dargestellt werden. Die Einträge dieser Liste können wiederum als interaktive Schaltflächen ausgeführt sein, bei deren Aktivierung die entsprechende Anpassung ungesetzt wird.

Zur Umsetzung einer Anpassung kann das Bereitstellungssystem 100 einen oder mehrere Steuerbefehle an ein betroffenes Endoskop-Aufbereitungsgerät senden, und/oder Anweisungen in Textform zur Wiedergabe an eine manuelle Aufbereitungsstation übermitteln.

## Patentansprüche

1. Bereitstellungssystem für Endoskope in einer Anwendungsumgebung mit einem oder mehreren Untersuchungsräumen (UR1, UR2, UR3) und einem oder mehreren Aufbereitungsplätzen (VR1, VR2, VR3, EDG1, EDG2, TS1, TS2, TS3, AS1, AS2) für Endoskope, wobei das Bereitstellungssystem eingerichtet ist,
- erste Informationen über vorgesehene Prozeduren zu empfangen, und
- zweite Informationen über einen Zustand eines oder mehrerer Endoskope zu empfangen, wobei die zweiten Informationen eine Angabe umfassen, ob ein Endoskop einsatzbereit ist, sich momentan in Verwendung befindet, oder momentan aufbereitet wird,
wobei das Bereitstellungssystem ferner eine Benutzerschnittstelle (104) mit einer grafischen Benutzeroberfläche (105, 205) umfasst,
**dadurch gekennzeichnet, dass** die grafische Benutzeroberfläche (105, 205)
- einen ersten Wiedergabebereich (110, 210) umfasst, in welchem Daten über einsatzbereite Endoskope angezeigt werden,
- einen zweiten Wiedergabebereich (120, 220) umfasst, in welchem Daten über geplante Prozeduren angezeigt werden, und
- einen dritten Wiedergabebereich (130, 230) umfasst, in welchem Daten über Endoskope angezeigt werden, die sich in einem Aufbereitungsprozess befinden.

2. Bereitstellungssystem nach Anspruch 1, **dadurch gekennzeichnet, dass** die zweiten Informationen zusätzlich Informationen über einen Aufenthaltsort des einen oder der mehreren Endoskope umfassen.

3. Bereitstellungssystem nach Anspruch 1 oder 2, **dadurch gekennzeichnet, dass** der erste Wiedergabebereich (110, 210), der zweite Wiedergabebereich (120, 220), und der dritte Wiedergabebereich (130, 230) entlang einer Hauptrichtung angeordnet sind, wobei der dritte Wiedergabebereich zwischen dem ersten Wiedergabebereich und dem zweiten Wiedergabebereich angeordnet ist.

4. Bereitstellungssystem nach Anspruch 3, **dadurch gekennzeichnet, dass** Daten für einzelne Endoskope und/oder Prozeduren in den Wiedergabebereichen (110, 120, 130, 210, 220, 230) entlang einer zweiten Hauptrichtung angeordnet sind, welche senkrecht zu der ersten Hauptrichtung verläuft.

5. Bereitstellungssystem nach Anspruch 4, **dadurch gekennzeichnet, dass** die Daten in dem ersten Wiedergabebereich (210), dem zweiten Wiedergabebereich (220), und dem dritten Wiedergabebereich (230) so angeordnet sind, dass Daten zu einem Endoskop, die im ersten Wiedergabebereich (210) oder im dritten Wiedergabebereich (230) dargestellt sind, entlang der zweiten Hauptrichtung mit Daten einer Prozedur im zweiten Wiedergabebereich (220) fluchten, in welcher das jeweilige Endoskop verwendet werden soll.

6. Bereitstellungssystem nach einem der vorangehenden Ansprüche, **dadurch gekennzeichnet, dass** im dritten Wiedergabebereich für jedes in einem Aufbereitungsprozess befindliche Endoskop eine Visualisierung (131, 231) dargestellt wird, welche den Fortschritt des Aufbereitungsprozesses wiedergibt.

7. Bereitstellungssystem nach Anspruch 6, **dadurch gekennzeichnet, dass** die Visualisierung (231) für jeden Schritt eines mehrere Schritte umfassenden Aufbereitungsprozesses ein Visualisierungselement (232) umfasst, welches anzeigt, ob der jeweilige Schritt abgeschlossen, in Arbeit, oder ausstehend ist.

8. Bereitstellungssystem nach einem der vorangehenden Ansprüche, **dadurch gekennzeichnet, dass** im dritten Wiedergabebereich (130, 230) für jedes in einem Aufbereitungsprozess befindliche Endoskop ein voraussichtlicher Zeitpunkt angezeigt wird, zu dem das Endoskop verfügbar sein wird.

9. Bereitstellungssystem nach einem der vorangehenden Ansprüche, **dadurch gekennzeichnet, dass** das Bereitstellungssystem eingerichtet ist, bei der Ermittlung eines Zeitpunktes, zu dem ein Endoskop voraussichtlich verfügbar sein wird, historische Daten vorausgegangener Aufbereitungsprozesse zu berücksichtigen.

10. Bereitstellungssystem nach Anspruch 9, **dadurch gekennzeichnet, dass** das Bereitstellungssystem eigerichtet ist, die Dauer von durchgeführten Aufbereitungsprozessen zu speichern und statistisch auszuwerten.

11. Verfahren zum Betrieb eines Bereitstellungssystems für Endoskope, mit den Schritten:
- Empfangen erster Informationen über geplante Prozeduren,
- Empfangen zweiter Informationen über einen Zustand eines oder mehrerer Endoskope, wobei die zweiten Informationen eine Angabe umfassen, ob ein Endoskop einsatzbereit ist, sich momentan in Verwendung befindet, oder momentan aufbereitet wird, und
- Anzeigen der ersten und zweiten Informationen in einer grafischen Benutzeroberfläche (105, 205),
**dadurch gekennzeichnet, dass** die grafische Benutzeroberfläche (105, 205)
- einen ersten Wiedergabebereich (110, 210) umfasst, in welchem Daten über einsatzbereite Endoskope angezeigt werden,
- einen zweiten Wiedergabebereich (120, 220) umfasst, in welchem Daten über geplante Prozeduren angezeigt werden, und
einen dritten Wiedergabebereich (130, 230) umfasst, in welchem Daten über Endoskope angezeigt werden, die sich in einem Aufbereitungsprozess befinden.

12. Verfahren nach Anspruch 11, **dadurch gekennzeichnet, dass** die zweiten Informationen zusätzlich Informationen über einen Aufenthaltsort des einen oder der mehreren Endoskope umfassen.
